(19) **Europäisches Patentamt**
**European Patent Office**
**Office européen des brevets**

(11) **EP 4 484 460 A1**

(12) **EUROPÄISCHE PATENTANMELDUNG**

(43) Veröffentlichungstag:
01.01.2025 Patentblatt 2025/01

(21) Anmeldenummer: 23182104.2

(22) Anmeldetag: 28.06.2023

(51) Internationale Patentklassifikation (IPC):
C08G 18/09 (2006.01)      C08G 18/79 (2006.01)
C08G 18/28 (2006.01)      C08G 18/32 (2006.01)
C08G 18/18 (2006.01)      C07D 251/34 (2006.01)

(52) Gemeinsame Patentklassifikation (CPC):
C08G 18/092; C07D 251/34; C08G 18/1875;
C08G 18/282; C08G 18/2825; C08G 18/3206;
C08G 18/792; C08G 2115/02; C08G 2150/00;
C08G 2170/00

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **Covestro Deutschland AG**
**51373 Leverkusen (DE)**

(72) Erfinder:
• **Kaese, Esther**
**51375 Leverkusen (DE)**

• **Munera Parra, Alejandro**
**51373 Leverkusen (DE)**
• **Kiecherer, Johannes**
**50969 Köln (DE)**
• **Golka, Leonie**
**50937 Köln (DE)**
• **Behrendt, Frank**
**40764 Langenfeld (DE)**
• **Heilmann, Alicia**
**42477 Radevormwald (DE)**

(74) Vertreter: **Levpat**
**c/o Covestro AG**
**Gebäude K12**
**51365 Leverkusen (DE)**

(54) **VERFAHREN ZUR HERSTELLUNG VON ISOCYANURATGRUPPEN-HALTIGEN POLYISOCYANATEN**

(57) Die Erfindung betrifft ein Verfahren zur Herstellung von Isocyanuratgruppen-haltigen Polyisocyanaten durch Trimerisierung

A) wenigstens eines organischen Di- oder Polyisocyanats mit unabhängig voneinander aliphatisch, cycloaliphatisch und/oder araliphatisch gebundenen Isocyanatgruppen,
in Gegenwart
B) wenigstens eines Trimerisierungskatalysators ausgewählt aus der Gruppe bestehend aus quaternären Tetraalkylammoniumhydroxiden, quaternären Trialkylarylammonium-hydroxiden und Hydroxyalkyl-substituierten quaternären Ammoniumhydroxiden vom Cholin-Typ und
C) wenigstens eines Alkohols als Lösemittel,
umfassend oder bestehend aus den folgenden Schritten
I) Vorlegen der Komponente A in einem Reaktor,
II) Zuführen der Gesamtmenge an Katalysatorkomponente B, sowie einer ersten Teilmenge der Komponente C ($C_{T1}$), und Trimerisierung der Komponente A bis zum Erhalt eines Trimerisierungsgrades $T_g$ im Bereich von 0,5 bis 25 %, bevorzugt von 0,5 bis 20 %, wobei gilt

$$T_g = (NCO_0 - NCO_t) / NCO_0,$$

wobei
$NCO_0$
der ursprünglich in der vorgelegten Komponente A vorhandenen Menge an freien NCO-Gruppen entspricht und
$NCO_t$
der Menge an freien NCO-Gruppen in der Reaktionslösung zum Zeitpunkt t entspricht,
jeweils bestimmt mittels NCO-Titration gemäß M105-ISO 11909,
III) Zuführen einer zweiten Teilmenge der Komponente C ($C_{T2}$) unter Fortführung der Trimerisierung der Komponente A.

**Beschreibung**

Die Herstellung Isocyanuratgruppen-haltiger Polyisocyanate durch Trimerisierung monomerer Di- und/oder Polyisocyanate ist seit langem bekannt.

Üblicherweise werden Trimerisierungskatalysatoren eingesetzt, wie beispielsweise quaternäre Tetraalkyl- oder Trialkylarylammoniumhydroxide, wie z.B. Triton-B und Hydroxyalkylsubstituierte quaternäre Ammoniumhydroxide vom Cholin-Typ, z.B. Cholinacetat.

Die bekannten Verfahren des Standes der Technik (z.B. EP 2 700 665 A1) werden so geführt, dass zunächst eine alkoholische Lösung des Katalysators der zu trimerisierenden Isocyanatkomponente zugeführt wird. Durch den dadurch bedingten Start der exothermen Reaktion steigt die Temperatur der Reaktionsmischung an. Durch Zugabe angepasster Mengen der Katalysatorlösung wird die Exothermie im weiteren Verlauf des Verfahrens kontrolliert.

Es wurde nun überraschend gefunden, dass das Reaktionsverhalten verbessert werden kann, wenn nach dem Start der Reaktion durch die anfänglichen Zugabe von alkoholischer Katalysatorlösung nur noch Alkohol, jedoch kein weiterer Katalysator mehr nachdosiert wird. Dieses Dosierungsverhalten führt dazu, dass bei gleichbleibender Reaktionsdauer weniger Katalysator verbraucht wird bzw. bei gleichbleibender Katalysatormenge die Reaktionsdauer durch einen schnelleren Reaktionsstart für Semi-batch Verfahren verkürzt und der Durchsatz für Kaskaden-Verfahren erhöht werden kann. Im ersten Fall wird durch den geringeren Verbrauch an Katalysator auch weniger Stopper benötigt, um die Reaktion abzustoppen, und im Produkt liegt weniger Katalysator vor, was die Produktqualität verbessert (weniger Tendenz zu Verfärbung). Eine reduzierte Reaktionsdauer bedeutet ein wirtschaftlicheres Verfahren. Darüber hinaus kann eine verbesserte Anpassung der Reaktionsführung an die Qualität des verwendeten Diisocyanats (in Bezug auf azide Komponenten) erfolgen, da zugegebene Katalysator- und Alkoholmengen unabhängig voneinander kontrolliert werden können.

Gegenstand der Erfindung ist ein Verfahren zur Herstellung von Isocyanuratgruppen-haltigen Polyisocyanaten durch Trimerisierung

A) wenigstens eines organischen Di- oder Polyisocyanats mit unabhängig voneinander aliphatisch, cycloaliphatisch und/oder araliphatisch gebundenen Isocyanatgruppen,
in Gegenwart
B) wenigstens eines Trimerisierungskatalysators ausgewählt aus der Gruppe bestehend aus quaternären Tetraalkylammoniumhydroxiden, quaternären Trialkylarylammonium-hydroxiden und Hydroxyalkyl-substituierten quaternären Ammoniumhydroxiden vom Cholin-Typ und
C) wenigstens eines Alkohols als Lösemittel,
umfassend oder bestehend aus den folgenden Schritten

I) Vorlegen der Komponente A in einem Reaktor,
II) Zuführen der Gesamtmenge an Katalysatorkomponente B, sowie einer ersten Teilmenge der Komponente C ($C_{T1}$), und Trimerisierung der Komponente A bis zum Erhalt eines Trimerisierungsgrades $T_g$ im Bereich von 0,5 bis 25 %, bevorzugt 0,5 bis 20 %, wobei gilt

$$T_g = (NCO_0 - NCO_t) / NCO_0,$$

wobei

$NCO_0$    der ursprünglich in der vorgelegten Komponente A vorhandenen Menge an freien NCO-Gruppen entspricht und

$NCO_t$    der Menge an freien NCO-Gruppen in der Reaktionslösung zum Zeitpunkt t entspricht,

jeweils bestimmt mittels NCO-Titration gemäß M105-ISO 11909,

III) Zuführen einer zweiten Teilmenge der Komponente C ($C_{T2}$) unter Fortführung der Trimerisierung der Komponente A.

Es ist ein wesentlicher Aspekt des Verfahrens, dass nach Schritt II keine weitere Zugabe an Katalysatorkomponente B erfolgt.

Im Rahmen der vorliegenden Erfindung werden unter aliphatischen Verbindungen solche verstanden, die ausschließlich offenkettige aliphatische Gruppen aufweisen, wobei diese verzweigt oder unverzweigt sein können. Cycloaliphatische Verbindungen sind solche, die mindestens ein cycloaliphatisches Ringsystem enthalten. Araliphatische Verbindungen sind solche, die mindestens eine araliphatische Gruppe aufweisen.

Zur Durchführung des erfindungsgemäßen Verfahrens können alle organischen Di- oder Polyisocyanate mit einem (mittleren) Molekulargewicht von 154 - 600 g/mol mit unabhängig voneinander aliphatisch, cycloaliphatisch und/oder araliphatisch gebundenen Isocyanatgruppen in reiner Form oder als beliebige Mischungen untereinander verwendet werden. Beispielhaft seien genannt: Pentamethylendiisocyanat (PDI), Hexamethylendiisocyanat (HDI), 2-Methylpentan-1,5-diisocyanat (MPDI), 1,3- sowie 1,4-Bis(isocyanatomethyl)cyclohexan (1,3- sowie 1,4-$H_6$-XDI), 1,3- sowie 1,4-Bis(isocyanatomethyl)benzol (1,3- sowie 1,4-XDI), 3(4)-Isocyanatomethyl-1-methylcyclo-hexylisocyanat (IMCI); Isophorondiisocyanat (IPDI), Bis(isocyanatomethyl)norbornan (NBDI), 4-Isocyanatomethyl-1,8-octandiisocyanat (Triisocyanatononan, TIN), 1,3-Bis(isocyanatomethyl)benzol, 1,3-Bis(2-isocyanatopropyl-2)benzol und Bis(4(2)-Isocyanatocyclohexyl)methan ($H_{12}$MDI, Desmodur® W, Produkt der Covestro AG). Hierbei ist es belanglos, nach welchen Verfahren die vorstehend genannten (Poly)isocyanate hergestellt werden, d.h. mit oder ohne Verwendung von Phosgen.

Bevorzugt wird das wenigstens eine organische Di- oder Polyisocyanat ausgewählt aus der Gruppe bestehend aus PDI, HDI, MPDI, 1,3- sowie 1,4-$H_6$XDI, 1,3- sowie 1,4-XDI und NBDI. Besonders bevorzugt ist HDI oder eine Mischung von HDI mit PDI, MPDI, 1,3- sowie 1,4-$H_6$XDI, 1,3- sowie 1,4-XDI und/oder NBDI.

In Schritt I kann die Komponente A unter reduziertem Druck und gegebenenfalls Wärmezufuhr entgast werden. Es ist bevorzugt Komponente A in Schritt I zu entgasen.

Als Katalysatorkomponente B wird im erfindungsgemäßen Verfahren bevorzugt wenigstens ein quaternäres Trialkylarylammoniumhydroxid und/oder wenigstens ein Hydroxyalkyl-substituiertes quaternäres Ammoniumhydroxid vom Cholin-Typ eingesetzt. Besonders bevorzugt wird wenigstens ein Benzyltrialkylammoniumhydroxid und/oder wenigstens ein Hydroxyalkyl-substituiertes quaternäres Ammoniumhydroxid vom Cholin-Typ eingesetzt. Insbesondere bevorzugt wird Benzyltrimethylammoniumhydroxid (Triton-B) und/oder 2-Hydroxyethyl-trimethyl-ammoniumacetat (Cholinacetat) eingesetzt.

Die Katalysatorkomponente B wird im allgemeinen in Mengen von 0,001 bis 2 Gew.-%, vorzugsweise von 0,001 bis 1 Gew.-% und besonders bevorzugt von 0,001 bis 0,2 Gew.-% bezogen auf die Menge der eingesetzten Isocyanatkomponente A verwendet.

Bevorzugt werden als Lösemittelkomponente C beliebige aliphatische und/oder cycloaliphatische Alkohole, bevorzugt aliphatische Alkohole, eingesetzt, wobei niedermolekulare Mono- oder Diole bevorzugt sind. Beispielhaft seien genannt: Methanol, Ethanol, Isopropanol, n-Butanol, 2-Ethylhexanol, 2-Ethylhexan-1,3-diol, 1,2-Dihydroxyethan, 1,2-Dihydroxypropan, 1,3- und 1,4-Dihydroxybutan, 1,6- und 2,5-Dihydroxyhexan, oder 2,2,4-Trimethyl-1,3-dihydroxypentan oder beliebige Gemische dieser Alkohole. Besonders bevorzugt sind Alkohole mit wenigstens einer primären Alkoholgruppe. Ganz besonders bevorzugt sind n-Butanol, 2-Ethylhexanol, 2-Ethylhexan-1,3-diol, 1,2-Dihydroxyethan, 1,2-Dihydroxypropan, 1,3- und 1,4-Dihydroxybutan, 1,6-Dihydroxyhexan, oder 2,2,4-Trimethyl-1,3-dihydroxypentan oder beliebige Gemische dieser Alkohole

Der wenigstens eine in Schritt II verwendete Alkohol C kann identisch mit oder unterschiedlich zu dem wenigstens einen in Schritt III verwendeten Alkohol sein.

In Schritt II wird Gesamtmenge an Katalysatorkomponente B und eine erste Teilmenge der Lösemittelkomponente C ($C_{T1}$) dem Reaktor zugeführt und Komponente A bis zum Erhalt eines Trimerisierungsgrades $T_g$ im Bereich von 0,5 bis 25 %, bevorzugt von 0,5 bis 20 %, trimerisiert.

Das Zuführen der Komponenten B und C erfolgt bevorzugt derart, dass wenigstens ein Teil der Gesamtmenge der Katalysatorkomponente B gelöst in wenigstens einem Teil der ersten Teilmenge der Lösemittelkomponente C vorliegt. Dabei sind folgende Ausführungsformen bevorzugt:

Die Gesamtmenge der Komponente B wird gelöst in der gesamten ersten Teilmenge der Komponente C dem Reaktor zugeführt (Ausführungsform a), wobei das Zuführen kontinuierlich oder diskontinuierlich erfolgen kann.

Die Gesamtmenge der Komponente B wird gelöst in einem ersten Teil der ersten Teilmenge der Komponente C dem Reaktor zugeführt und der restliche Teil der ersten Teilmenge der Komponente C wird als reine Komponente C dem Reaktor zugeführt (Ausführungsform b), wobei beides unabhängig voneinander kontinuierlich oder diskontinuierlich erfolgen kann. Der Ausdruck "reine Komponente C" bedeutet in diesem Zusammenhang, das C keine Katalysatoren der Komponente B enthält.

Die Katalysatorkomponente B wird in Mengen von bevorzugt 0,3 bis 8 Gew.-%, besonders bevorzugt 0,5 bis 5 Gew.-%, ganz besonders bevorzugt 0,8 bis 2 Gew.-% verwendet, bezogen auf die in Schritt II eingesetzte Gesamtmenge an Lösemittelkomponente C, d.h. bezogen auf $C_{T1}$.

Schritt II kann so geführt werden, dass die Trimerisierung bereits während oder aber erst nach dem Zuführen der Komponente B startet. Dies kann thermisch beeinflusst werden. Die erstgenannte Möglichkeit ist bevorzugt.

Die Trimerisierung in Schritt II wird bei Reaktionstemperaturen von bevorzugt 50 bis 120 °C, besonders bevorzugt 55 bis 90 °C. Wird HDI als Komponente A verwendet wird die Trimerisierung in Schritt II ganz besonders bevorzugt bei 57 bis 65 °C durchgeführt.

In Schritt III wird dem Reaktor eine zweite Teilmenge der Lösemittelkomponente C ($C_{T2}$) zugeführt. Dies kann kontinuierlich oder diskontinuierlich erfolgen.

Die Gesamtmenge an zugeführter Lösemittelkomponente C entspricht der Summe der beiden Teilmengen aus den Schritten II und III ($C_{T1}$ + $C_{T2}$). Die Gesamtmenge an zugeführter Lösemittelkomponente C beträgt bevorzugt 0,3 bis 5 Gew.-%, besonders bevorzugt 1 bis 2 Gew.-%, jeweils bezogen auf die Menge der eingesetzten Isocyanatkomponente A.

Die Trimerisierung in Schritt III erfolgt bei Reaktionstemperaturen von bevorzugt 50 bis 120 °C, besonders bevorzugt 55 bis 90 °C. Wird HDI als Komponente A verwendet wird die Trimerisierung in Schritt II ganz besonders bevorzugt bei 60 bis 65 °C durchgeführt.

Die Reaktionstemperaturen in Schritt II und Schritt III können gleich sein oder sich unterscheiden.

In Schritt III wird die Trimerisierung der Komponente A fortgeführt bis der gewünschte Trimerisierungsgrad erreicht ist.

Die Reaktion kann dann abgestoppt werden, wobei dies chemisch oder thermisch erfolgen kann. Bevorzugt ist eine thermische Abstoppung. Im Falle einer chemischen Abstoppung wird die Reaktionslösung durch Zugabe einer sauren Verbindung, einer Säure und/oder eines Alkylierungsmittels abgestoppt. Die bevorzugte thermische Abstoppung erfolgt entweder bei der gewählten Reaktionstemperatur durch einfaches Nachrühren nach Abklingen der Reaktion oder aber durch Temperaturerhöhung um bis zu 50 °C und Nachrühren bei dieser Temperatur. Bevorzugt wird die Abstopptemperatur um bis zu 20 °C gegenüber der Reaktionstemperatur leicht erhöht. Besonders bevorzugt wird die thermische Abstoppung bei Reaktionstemperatur durchgeführt.

Das erfindungsgemäße Verfahren kann als Batch-Verfahren, Semi-Batch-Verfahren oder kontinuierlich, in einem oder mehreren Rührkesseln, durchgeführt werden.

Nach Beendigung der Trimerisierungsreaktion, gegebenenfalls durch Abstoppung, liegt als Reaktionsprodukt eine Lösung des Isocyanuratgruppen-haltigen Polyisocyanats in überschüssigem monomerem Di- und/oder Polyisocyanat der Komponente A vor.

In einer bevorzugten Ausführungsform der Erfindung wird in einem nachgeschalteten Schritt von diesem Reaktionsgemisch noch vorhandenes monomeres Di- und/oder Polyisocyanat der Komponente A destillativ abgetrennt. Dies erfolgt vorzugsweise durch Dünnschichtdestillation im Vakuum, beispielsweise bei einem Druck von unter 1,0 mbar, vorzugsweise unter 0,5 mbar, besonders bevorzugt unter 0,2 mbar, unter möglichst schonenden Bedingungen, beispielsweise bei einer Temperatur von 100 bis 200 °C, vorzugsweise von 120 bis 180 °C. Die Abtrennung des monomeren Di- und/oder Polyisocyanats kann einstufig, bevorzugt aber mehrstufig erfolgen. So wird beispielsweise ein Fallfilmverdampfer als Vorverdampfer eingesetzt, bei dem die Hauptmenge des monomeren Di- und/oder Polyisocyanats abgetrennt wird, im nachgeschalteten Dünnschichtverdampfer erfolgt die Abtrennung weiteren Ausgangsisocyanats. Auf diese Weise erhält man hochwertige Isocyanuratgruppen-haltige Polyisocyanate, die einen Gehalt an freiem monomeren Di- und/oder Polyisocyanat von maximal 0,5 Gew.-%, vorzugsweise von maximal 0,1 Gew.-%, aufweisen. Die anfallenden Destillate werden erneut zur Trimerisierung eingesetzt.

In einer weiteren Ausführungsform werden die monomeren Di- und/oder Polyisocyanate durch Extraktion mit geeigneten, gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Reaktionsprodukt abgetrennt. Dieses Verfahren ist weniger bevorzugt.

Die so erhaltenen monomerenarmen Isocyanuratgruppen-haltige Polyisocyanate werden als solche verwendet oder aber in geeigneten gegenüber NCO-Gruppen inerten Lösungsmitteln zu Polyisocyanat-Lösungen gelöst. Die nach dem erfindungsgemäßen Verfahren hergestellten Polyisocyanate werden in den bekannten Anwendungen wie z. B. 2-Komponenten Polyurethanlacken oder in Klebstoffanwendungen verwendet. Die so erhaltenen Polyisocyanate dienen darüber hinaus wie es bekannt ist als Ausgangsstoffe für weitere daraus hergestellte Derivate wie z. B. blockierte Polyisocyanate oder hydrophilierte Polyisocyanate.

**Experimenteller Teil:**

Verwendete Edukte wurden sofern nicht anders beschrieben ohne weitere Aufreinigung eingesetzt. Hexamethylendiisocyanat und Desmodur LD wurden von der Covestro Deutschland AG bezogen. Alle anderen Edukte wurden bei Sigma Aldrich (Merck AG) bestellt: Triton B 40% in Methanol, 2-Ethylhexanol, n-Butanol, 2-Ethylhexan-1,3-diol, 4-Heptanol und Cholinacetat.

Es kamen folgende Standard-Methoden zum Einsatz:

NCO-Werte wurden mittels NCO-Titration gemäß M105-ISO 11909 bestimmt.
Viskositäten wurden gemäß M014-ISO 3219/A.3 bestimmt.
Der freie Monomer-Gehalt wurde mittels M106-ISO 10283 bestimmt.

Die online Reaktionsverfolgung wurde mithilfe von Raman Spektroskopie durchgeführt. Dazu wurde das Gerät RAMAN RXN2 der Fa. Kaiser optics verwendet und der dazugehörige Messsensor in das Reaktionsbehältnis mit eingeführt und alle 2 Minuten ein Spektrum aufgenommen. Der Bande bei Wellenzahl 1760 cm$^{-1}$ kann die C=O-Bande des Isocyanurats eindeutig zugeordnet werden. In Kombination mit der NCO-Bestimmung mittels Titration und mit der Annahme, dass alle abreagierten NCO-Gruppen in das Isocyanurat einfließen, wurde eine Kalibrierung der Signale vorgenommen.

**Aufbau der Anlage**

Die Anlage besteht aus einem 0,5 Liter Doppelmantel-Glasreaktor (Fa. Büchi, Typ 2), welcher bis zu einem Betriebsdruck von 6 bar und einer Betriebstemperatur von 200 °C betrieben werden kann. Die Beheizung des Reaktorinnenraums erfolgt über ein an den Doppelmantel angeschlossenes Thermostat (Fa. Huber, Typ Ministat 240). Die Temperaturregelung im Thermostat erfolgt anhand der im Reaktorinnenraum mit einem Thermoelement gemessenen Temperatur. Eine möglichst homogene Durchmischung im Reaktor wird mit einem über den Reaktordeckel eingelassenen Rührer gewährleistet. Über den Reaktordeckel kann eine Raman-Sonde in den Reaktor eingeführt und zur Online Reaktionsverfolgung genutzt werden. Zudem besteht die Möglichkeit aus dem Reaktor mittels einer Spritze Proben für Offline-Analysen zu entnehmen.

**Batch-Reaktion**

Beispiel 1: Referenzversuch mit herkömmlicher Dosierung (nicht erfindungsgemäß)

In einem 0,5 L Rührreaktor wurden 350 g Hexamethylendiisocyanat (HDI) vorgelegt und auf 70 °C erwärmt, um das Diisocyanat bei dieser Temperatur für 60 min bei 20 mbar zu entgasen. Nach Belüften des Reaktionsbehälters und Abkühlen des Diisocyanats auf 60 °C wurden 4,6 g einer 0,5 Gew.-% Triton B Lösung in 2-Ethylhexnol unter Rührern (1000 rpm) möglichst schnell in den Behälter dosiert. Nach einer kurzen Karenzphase, trat der Reaktionsstart ein, der zu einer Abnahme des freien NCO-Werts führte. Ein Trimerisierungsgrad von 10% wurde zügig erreicht. Im weiteren Zeitverlauf von 3 Stunden nahm der NCO-Wert weiter ab, wenn auch mit einer geringeren Abnahmerate als zuvor.

Beispiel 2: Anwendung des beschriebenen Verfahrens (erfindungsgemäß)

Das unter Beispiel 1 beschriebene Experiment wurde identisch wiederholt bis ein Trimerisierungsgrad von 20% erreicht war. Dann erfolgte die Zugabe von 1,16 g 2-Ethylhexanol als eine Portion. Nach einer kurzen Wartezeit fiel der NCO-Wert stärker ab als im Referenzexperiment (Beispiel 1). Die Abnahme des NCO-Werts ging über das Maß heraus, was durch die Reaktion von freien NCO-Gruppen mit dem zugegeben Alkohol zur Bildung von Urethanen und Allophanaten zu erklären ist. Die erhöhte Anzahl an Isocyanurateinheiten im Vergleich zu Beispiel 1 konnte mittels Raman-Spektroskopie bestätigt werden.
Vergleichbare Experimente wurden ebenso mit n-Butanol, 2-Ethylhexan-1,3-diol und 4-Heptanol durchgeführt. Ebenso wurde die Kombination Cholinacetat/2-Ethylhexanol im Experiment bestätigt.

Beispiel 3: Die besondere Rolle des Alkohols im Vergleichsexperiment (nicht erfindungsgemäß)

Das unter Beispiel 1 beschriebene Experiment wurde identisch wiederholt bis ein Trimerisierungsgrad von 20% erreicht war. Dann erfolgte die Zugabe von 5 g Desmodur LD (Covestro Verkaufsbezeichnung von 2-Ethylhexyl (6-isocyanathexyl)-carbamat) als eine Portion. Im Vergleich zum Referenzbeispiel 1 erhöhte sich der NCO-Wert minimal, weil die zugegebene Komponenten Desmodur LD freie Isocyanatgruppen beinhaltet. Davon abgesehen, ergab sich kein anderer Reaktionsverlauf und es wurde keine erhöhte Anzahl an Isocyanurateinheiten gebildet.

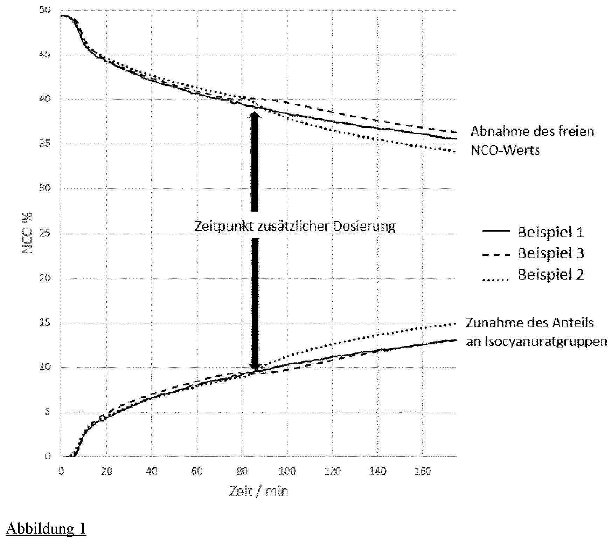

In Abbildung 1 sind die Reaktionsverläufe der Beispiele 1-3 vergleichend dargestellt.

Beschreibung der Abbildung 1:

Beispiel 1 = durchgezogene Linie; Beispiel 2 = gepunktete Linie; Beispiel 3 = gestrichelte Linie. Vergleich von 3 Trimerisierungsreaktionen bei 60 °C mit Triton B als Katalysator und 2-Ethylhexanol als Alkohol. Die Pfeile weisen auf den Zeitpunkt von zusätzlichen Dosierungen für Beispiel 2 und 3 hin.

**Semi-batch Versuche (Reduzierung der Reaktionszeit)**

Beispiel 4: Semi-batch Versuch mit herkömmlicher Dosierung (nicht erfindungsgemäß)

In einem 0,5 L Rührreaktor wurden 350 g Hexamethylendiisocyanat (HDI) vorgelegt und auf 70 °C erwärmt, um das Diisocyanat bei dieser Temperatur für 60 min bei 1 mbar zu entgasen. Nach Belüften des Reaktionsbehälters und Abkühlen des Diisocyanats auf 65 °C wurde die Dosierung einer 1,5 Gew.-% Triton B Lösung in 2-Ethylhexnol unter Rühren (500 rpm) mit 0,18 ml/min in den Behälter gestartet. Nach 10 min wurde die Zugabe der Katalysatorlösung gestoppt. Nach weiteren 30 min wurde die Zugabe von Katalysatorlösung mit 0,017 ml/min wieder aufgenommen bis insgesamt 1,85 g Katalysatorlösung in den Behälter dosiert wurden. Die Reaktion wurde bis zu einem Trimerisierungsgrad von 11,2% fortgesetzt. Nun erfolgte das Stoppen der Reaktion durch Zugabe von DBP (Dibutylphosphat; 120 Gew.-% in Bezug auf die eingesetzte Menge Triton B).

Beispiel 5: Semi-batch Versuch mit Anwendung des beschriebenen Verfahrens (erfindungsgemäß)

In einem 0,5 L Rührreaktor wurden 350 g Hexamethylendiisocyanat (HDI) vorgelegt und auf 70 °C erwärmt, um das Diisocyanat bei dieser Temperatur für 60 min bei 1 mbar zu entgasen. Nach Belüften des Reaktionsbehälters und Abkühlen des Diisocyanats auf 65 °C wurde die Dosierung einer 1,9 Gew.-% Triton B Lösung in 2-Ethylhexnol unter Rühren (500 rpm) mit 0,18 ml/min in den Behälter gestartet. Nach 10 min wurde die Zugabe der Katalysatorlösung gestoppt. Nach weiteren 30 min, bei $T_g$ = 7,6%, wurde die Zugabe von reinem Alkohol mit 0,017 ml/min wieder aufgenommen bis insgesamt 1,85 g bestehend aus Katalysatorlösung und zugegebenen Alkohol in den Behälter dosiert wurden. Die Reaktion wurde bis zu einem Trimerisierungsgrad von 11,2% fortgesetzt. Nun erfolgte das Stoppen der Reaktion durch Zugabe von DBP (Dibutylphosphat; 120 Gew.-% in Bezug auf die eingesetzte Menge Triton B).

Tabelle 1: Vergleich von Beispiel 4 und 5 in Bezug auf die Reaktionszeiten:

Tabelle 1

| Trimerisierungsgrad | 0 % (Beginn des Versuchs) | 11,2 % (Zielwert des Versuchs) |
|---|---|---|
|  | Karenzphase in min | Zielwert erreicht nach x min |
| Beispiel 4 | 12 | 122 |
| Beispiel 5 | 4 | 64 |

Wie aus Tabelle 1 ersichtlich, startet im erfindungsgemäßen Fall die Reaktion schneller und die Reaktionsdauer wird verkürzt.

Die in Beispiel 4 und 5 erzeugten Rohwaren wurden auf dieselbe Weise destilliert, um den Überschuss an freiem HDI-Monomer zu entfernen. Wie Tabelle 2 zeigt, ist die erhaltene Produktqualität identisch (wie bei Einsatz der gleichen Katalysator- und Alkoholmengen zu erwarten).

Tabelle 2: Vergleich von Beispiel 4 und 5 in Bezug auf die Spezifikationen der nach Destillation erhaltenen Harze:

Tabelle 2

|  | Beispiel 4 | Beispiel 5 |
|---|---|---|
| NCO-Wert in % | 22,8 | 22,7 |
| Viskosität / mPas @ 23 °C | 1300 | 1200 |
| Freier Monomer-Gehalt | 0,03 | 0,04 |

**Patentansprüche**

1.    Verfahren zur Herstellung von Isocyanuratgruppen-haltigen Polyisocyanaten durch Trimerisierung

A) wenigstens eines organischen Di- oder Polyisocyanats mit unabhängig voneinander aliphatisch, cycloaliphatisch und/oder araliphatisch gebundenen Isocyanatgruppen, in Gegenwart

B) wenigstens eines Trimerisierungskatalysators ausgewählt aus der Gruppe bestehend aus quaternären Tetraalkylammoniumhydroxiden, quaternären Trialkylarylammonium-hydroxiden und Hydroxyalkyl-substituierten quaternären Ammoniumhydroxiden vom Cholin-Typ
und

C) wenigstens eines Alkohols als Lösemittel,

umfassend oder bestehend aus den folgenden Schritten

I) Vorlegen der Komponente A in einem Reaktor,
II) Zuführen der Gesamtmenge an Katalysatorkomponente B, sowie einer ersten Teilmenge der Komponente C ($C_{T1}$), und Trimerisierung der Komponente A bis zum Erhalt eines Trimerisierungsgrades $T_g$ im Bereich von 0,5 bis 25 %, bevorzugt von 0,5 bis 20 %, wobei gilt

$$T_g = (NCO_0 - NCO_t) / NCO_0,$$

wobei

$NCO_0$ der ursprünglich in der vorgelegten Komponente A vorhandenen Menge an freien NCO-Gruppen entspricht und
$NCO_t$ der Menge an freien NCO-Gruppen in der Reaktionslösung zum Zeitpunkt t entspricht,

jeweils bestimmt mittels NCO-Titration gemäß M105-ISO 11909,
III) Zuführen einer zweiten Teilmenge der Komponente C ($C_{T2}$) unter Fortführung der Trimerisierung der Komponente A.

2. Verfahren gemäß Anspruch 1, wobei das wenigstens eine organischen Di- oder Polyisocyanat (A) ausgewählt ist aus der Gruppe bestehend aus PDI, HDI, MPDI, 1,3- sowie 1,4-$H_6$XDI, 1,3-sowie 1,4-XDI und NBDI.

3. Verfahren gemäß Anspruch 1 oder 2, wobei als Komponente B wenigstens ein Benzyltrialkylammoniumhydroxid und/oder wenigstens ein Hydroxyalkyl-substituiertes quaternäres Ammoniumhydroxid vom Cholin-Typ eingesetzt wird.

4. Verfahren gemäß Anspruch 3, wobei Benzyltrimethylammoniumhydroxid (Triton-B) und/oder 2-Hydroxyethyl-trimethyl-ammoniumacetat (Cholinacetat) eingesetzt wird.

5. Verfahren gemäß einem der Ansprüche 1 bis 4, wobei als Komponente C aliphatische und/oder cycloaliphatische Mono- oder Diole eingesetzt werden, bevorzugt solche mit wenigstens einer primären Alkoholgruppe.

6. Verfahren gemäß einem der Ansprüche 1 bis 5, wobei Katalysatorkomponente B in Mengen von 0,001 bis 2 Gew.-%, bevorzugt von 0,001 bis 1 Gew.-% und besonders bevorzugt von 0,001 bis 0,2 Gew.-%, jeweils bezogen auf die Menge der eingesetzten Isocyanatkomponente A, verwendet.

7. Verfahren gemäß einem der Ansprüche 1 bis 6, wobei die Katalysatorkomponente B in Mengen von 0,3 bis 8 Gew.-%, bevorzugt 0,5 bis 5 Gew.-%, besonders bevorzugt 0,8 bis 2 Gew.-%, bezogen auf die in Schritt II eingesetzte erste Teilmenge der Komponente C ($C_{T1}$), eingesetzt wird.

8. Verfahren gemäß einem der Ansprüche 1 bis 7, wobei die Trimerisierung in Schritt II bei Reaktionstemperaturen von 50 bis 120 °C, bevorzugt 55 bis 90 °C durchgeführt wird.

9. Verfahren gemäß einem der Ansprüche 1 bis 8, wobei die in Schritt II und III zugeführte Gesamtmenge an Lösemittelkomponente C ($C_{T1} + C_{T2}$) 0,3 bis 5 Gew.-%, bevorzugt 1 bis 2 Gew.-%, jeweils bezogen auf die Menge der eingesetzten Isocyanatkomponente A, beträgt.

10. Verfahren gemäß einem der Ansprüche 1 bis 9, wobei die Trimerisierung in Schritt III bei Reaktionstemperaturen von 50 bis 120 °C, bevorzugt 55 bis 90 °C durchgeführt wird.

11. Verfahren gemäß einem der Ansprüche 1 bis 10, wobei das Verfahren als Batch-Verfahren, Semi-Batch-Verfahren oder kontinuierlich, in einem oder mehreren Rührkesseln, durchgeführt wird.

12. Verfahren gemäß einem der Ansprüche 1 bis 11, wobei die Trimerisierung der Komponente A beim Erreichen eines gewünschten Trimerisierungsgrades chemisch oder thermisch, bevorzugt thermisch, abgestoppt wird.

13. Verfahren gemäß einem der Ansprüche 1 bis 11, **dadurch gekennzeichnet, dass** in einem nachgeschalteten Schritt noch vorhandenes monomeres Di- und/oder Polyisocyanat der Komponente A destillativ vom Reaktionsprodukt abgetrennt wird.

Abbildung 1

Europäisches Patentamt

European Patent Office

Office européen des brevets

# EUROPÄISCHER RECHERCHENBERICHT

**Nummer der Anmeldung**

EP 23 18 2104

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 2 700 665 A1 (BAYER MATERIALSCIENCE AG [DE]) 26. Februar 2014 (2014-02-26) * Absätze [0001], [0010] – [0018], [0022] – [0030] * * Absatz [0046]; Beispiele 1,3 * ----- | 1-13 | INV. C08G18/09 C08G18/79 C08G18/28 C08G18/32 C08G18/18 C07D251/34 |
| A | EP 4 159 781 A1 (WANHUA CHEMICAL GROUP CO LTD [CN]; WANHUA CHEMICAL NINGBO CO LTD [CN]) 5. April 2023 (2023-04-05) * Absätze [0001] – [0009], [0013], [0035] – [0038], [0045] * * Absatz [0091]; Beispiele 1-3 * ----- | 1-13 | |
| A | JP 2023 083907 A (ASAHI KASEI CORP) 16. Juni 2023 (2023-06-16) * das ganze Dokument * ----- | 1-13 | |

| RECHERCHIERTE SACHGEBIETE (IPC) |
|---|
| C08G |

Der vorliegende Recherchenbericht wurde für alle Patentansprüche erstellt

| Recherchenort | Abschlußdatum der Recherche | Prüfer |
|---|---|---|
| München | 15. November 2023 | Neugebauer, Ute |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 23 18 2104

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-11-2023

| Im Recherchenbericht angeführtes Patentdokument | Datum der Veröffentlichung | Mitglied(er) der Patentfamilie | Datum der Veröffentlichung |
|---|---|---|---|
| EP 2700665 A1 | 26-02-2014 | CN 103626955 A | 12-03-2014 |
| | | EP 2700665 A1 | 26-02-2014 |
| | | HU E041618 T2 | 28-05-2019 |
| | | US 2014058102 A1 | 27-02-2014 |
| EP 4159781 A1 | 05-04-2023 | EP 4159781 A1 | 05-04-2023 |
| | | JP 2023534679 A | 10-08-2023 |
| | | KR 20220123439 A | 06-09-2022 |
| | | US 2023272151 A1 | 31-08-2023 |
| | | WO 2022061704 A1 | 31-03-2022 |
| JP 2023083907 A | 16-06-2023 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2700665 A1